(19) Europäisches Patentamt
European Patent Office
Office européen des brevets

(11) Veröffentlichungsnummer: **0 137 355 B1**

(12) **EUROPÄISCHE PATENTSCHRIFT**

(45) Veröffentlichungstag der Patentschrift: **20.11.86**

(51) Int. Cl.⁴: **C 07 C 87/34**, C 07 C 85/04

(21) Anmeldenummer: **84111047.1**

(22) Anmeldetag: **17.09.84**

(54) **2,2-Dichloro-3,3-dimethylcyclopropylmethylamin.**

(30) Priorität: **26.09.83 JP 176508/83**

(43) Veröffentlichungstag der Anmeldung:
**17.04.85 Patentblatt 85/16**

(45) Bekanntmachung des Hinweises auf die Patenterteilung:
**20.11.86 Patentblatt 86/47**

(84) Benannte Vertragsstaaten:
**AT BE CH DE FR GB IT LI NL**

(56) Entgegenhaltungen:
**US - A - 4 275 238**

**CHEMICAL ABSTRACTS, Band 90, Nr. 5, Januar 1979, Columbus, Ohio, USA, K. GASS, "Herbicidal triazine compounds", Seite 494, Zusammenfassung-Nr. 38 967 s**
**CHEMICAL ABSTRACTS, Band 91, Nr. 11, September 1979, Columbus, Ohio, USA, K. STEINBECK, "1-Bromomethyl-2,2-dichloropropane as an alkylation reagent", Seite 738, Zusammenfassung-Nr. 91 200h**

(73) Patentinhaber: **NIHON TOKUSHU NOYAKU SEIZO K.K., No.4, 2-chome, Nihonbashi Honcho Chuo-ku, Tokyo 103 (JP)**

(72) Erfinder: **Shiokawa, Kozo, 210-6, Shukugawara Tama-ku, Kawasaki-shi Kanagawa-ken (JP)**
Erfinder: **Kagabu, Shinzo, 432-131-105, Terada-machi, Hachioji-shi Tokyo (JP)**
Erfinder: **Sakawa, Shinji, 1-14-13, Tamadaira, Hino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

**EP 0 137 355 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft 2,2-Dichloro-3,3-dimethylcyclopropylmethylamin, eine neue Verbindung, die als Fungizid für Landwirtschaft und Gartenbau oder als synthetische Zwischenstufe für andere Cyclopropyl-Derivate und andere Verbindungen wertvoll ist, sowie ein Verfahren zur Herstellung dieser Verbindung.

Im einzelnen betrifft die Erfindung 2,2-Dichloro-3,3-dimethylcyclopropylmethylamin der nachstehenden Formel (I)

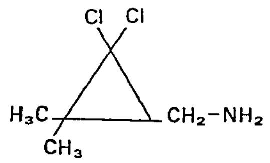

(I)

Die Verbindung der Formel (I) gemäss der vorliegenden Erfindung kann beispielsweise mit Hilfe des folgenden Verfahrens hergestellt werden, auf das sich die Erfindung ebenfalls erstreckt.

Ein Verfahren zur Herstellung von 2,2-Dichloro-3,3-dimethylcyclopropylmethylamin umfasst die Reaktion einer Verbindung der nachstehenden Formel (II)

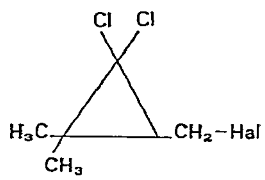

(II)

in der Hal ein Halogen-Atom bezeichnet, mit Ammoniak.

Untersuchungen seitens der Anmelderin haben ergeben, dass das vorstehende, in der bekannten Literatur nicht beschriebene 2,2-Dichloro-3,3-dimethylcyclopropylmethylamin synthetisiert werden kann und dass diese Verbindung als Zwischenprodukt für die Herstellung neuer Verbindungen mit biologischer Aktivität als Agrochemikalien, insbesondere beispielsweise einer Aktivität in bezug auf die Bekämpfung der Brusone-Krankheit von Reis, wie des N-2,2-Dichloro-3,3-dimethylcyclopropylmethylphenylacetamids und des N-2,2-Dichloro-3,3-dimethylcyclopropylmethylbenzamids wertvoll ist.

Weiterhin wurde gefunden, dass die Verbindung der vorliegenden Erfindung nicht nur als Zwischenprodukt für die Herstellung anderer Cyclopropyl-Derivate, wie sie oben beispielhaft angegeben sind, von Wert ist, sondern dass sie selbst eine hervorragende Bekämpfungswirkung gegen die Brusone-Krankheit von Reis zeigt.

Dementsprechend ist es Ziel der vorliegenden Erfindung, 2,2-Dichloro-3,3-dimethylcyclopropylmethylamin, eine neue Verbindung, sowie ein Verfahren zu seiner Herstellung verfügbar zu machen.

Die Verbindung gemäss der vorliegenden Erfindung kann beispielsweise mit Hilfe des folgenden Verfahrens hergestellt werden

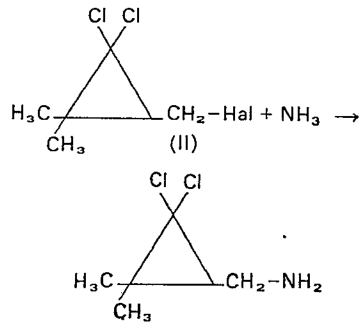

(II)

worin Hal ein Halogen-Atom bezeichnet.

In dem vorstehenden Reaktionsschema steht Hal speziell für ein Halogen-Atom wie Fluor, Chlor, Brom und Iod, vorzugsweise Chlor und Brom.

In dem durch das vorstehende Reaktionsschema dargestellten Verfahren zur Herstellung der Verbindung gemäss der vorliegenden Erfindung sind spezielle Beispiele für die Ausgangsverbindung der Formel (II) 2,2-Dichloro-3,3-dimethylcyclopropylmethylchlorid und 2,2-Dichloro-3,3-dimethylcyclopropylmethylbromid.

Das Verfahren der vorliegenden Erfindung wird speziell durch das folgende Beispiel beschrieben:

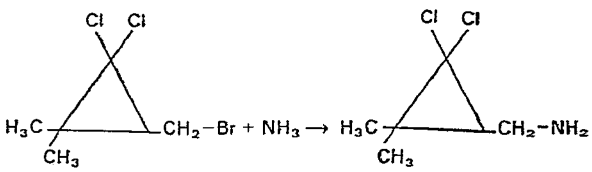

Zweckmässigerweise kann das Verfahren zur Herstellung der vorstehenden Verbindung gemäss der vorliegenden Erfindung unter Verwendung eines Lösungsmittels oder Verdünnungsmittels durchgeführt werden. Zu diesem Zweck können sämtliche inerten Lösungsmittel und Verdünnungsmitel verwendet werden.

Zu Beispielen für solche Lösungsmittel oder Verdünnungsmittel zählen Wasser; aliphatische, alicyclische und aromatische Kohlenwasserstoffe (ggf. chloriert) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Diisopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Die obige Reaktion kann in Gegenwart eines säurebindenden Mittels durchgeführt werden. Zu Beispielen für das säurebindende Mittel zählen die Hydroxide, Carbonate, Hydrogencarbonate und Alkoholate von Alkalimetallen, die allgemein verwendet werden, und tertiäre Amine wie Triethylamin, Diethylanilin und Pyridin.

Das Verfahren gemäss der vorliegenden Erfindung kann in einem weiten Temperaturbereich durchgeführt werden. Im allgemeinen kann es bei einer Temperatur zwischen etwa −20° C und dem Siedepunkt der Mischung, vorzugsweise zwischen etwa 0° C und etwa 100° C, durchgeführt werden. Die Reaktion wird zweckmässigerweise unter Atmosphärendruck durchgeführt, jedoch ist es auch möglich, unter erhöhtem oder vermindertem Druck zu arbeiten.

Die folgenden Beispiele erläutern die vorliegende Erfindung. Es ist jedoch darauf hinzuweisen, dass die Erfindung nicht auf diese Beispiele allein beschränkt ist.

*Beispiel 1 (Synthesebeispiel)*

2,2-Dichloro-3,3-dimethylcyclopropylmethylbromid (75 g) wurde in Ethanol (200 ml) gelöst, und Ammoniak (70 g) wurde hinzugefügt. Die Mischung wurde in einem Bombenrohr auf 90° C bis 100° C unter 40,5 bar (40 atm) 20 h erhitzt. Die Reaktionsmischung wurde abgekühlt, und das überschüssige Ammniak-Gas wurde entfernt. Dann wurde die Mischung in Hexan (1 l) gegossen. Die organische Schicht wurde mit 3 Anteilen von jeweils 200 ml Wasser gewaschen und dann getrocknet. Das Hexan wurde abgedampft, und die verbleibende Flüssigkeit wurde destilliert, wonach 50 g der gewünschten Verbindung gemäss der Erfindung, 2,2-Dichloro-3,3-dimethylcyclopropylmethylamin, mit einem Sdp. von 101-103° C/26,7 mbar (20 mmHg) erhalten wurden.

Das folgende Bezugsbeispiel erläutert die Synthese einer neuen, biologisch aktiven Verbindung, hergestellt aus der Verbindung gemäss der vorliegenden Erfindung als Zwischenprodukt.

*Bezugsbeispiel*

2,2-Dichloro-3,3-dimethylcyclopropylmethylamin (3,34 g) und Pyridin (2,0 g) wurden in Toluol (30 ml) gelöst, und unter Eiskühlung wurde eine Lösung von Chloroacetylchlorid (2,24 g) in 20 ml Toluol tropfenweise hinzugefügt. Die Mischung wurde 5 h gerührt, und die Reaktionsmischung wurde in Wasser gegossen. Die Toluol-Schicht wurde zweimal mit einer 3-proz. wässrigen Salzsäure, zweimal mit einer gesättigten wässrigen Natriumhydrogencarbonat-Lösung und danach mit Wasser gewaschen und anschliessend getrocknet. Das Toluol wurde unter vermindertem Druck abgedampft, und der Rückstand wurde mit Hexan gewaschen, wonach 4,85 g 2,2-Dichloro-3,3-dimethylcyclopropylmethylchloroacetylamid mit dem Schmp. 66° C erhalten wurden.

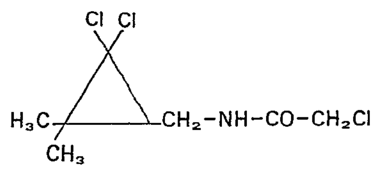
(D-1)

Mittels nahezu des gleichen Verfahrens wie in dem Bezugsbeispiel wurden die folgenden biologisch aktiven Verbindungen synthetisiert.

*Verbindung Nr.*

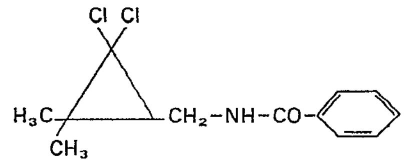

D-2
(Derivat) (Schmp. 96° C)

*Verbindung Nr.*

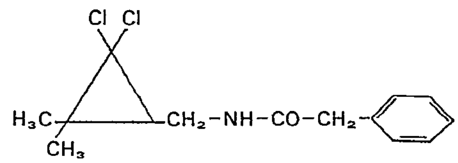

D-3
(Derivat) (Schmp. 75° C)

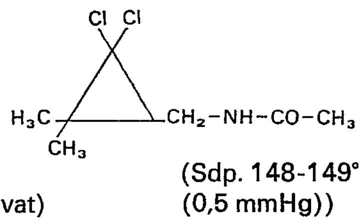

D-4 (Sdp. 148-149° C/0,67 mbar
(Derivat) (0,5 mmHg))

*Biologisches Test-Beispiel*

Test auf Wirksamkeit gegen die Brusone-Krankheit von Reis bei Wasseroberflächen-Anwendung:

*Herstellung einer Test-Verbindung:*

Wirkstoff: 50 Gew.-Teile;

Träger: 45 Gew.-Teile einer Mischung (1:5) aus Diatomeenerde und Kaolin;

Emulgator: 5 Gew.-Teile Polyoxyethylenalkylphenylether.

Die aktive Verbindung, der Träger und der Emulgator wurden in den angegebenen Mengen miteinander vermischt und pulverisiert, wodurch ein benetzbares Pulver gebildet wurde. Eine vorher festgelegte Menge des benetzbaren Pulvers wurde mit Wasser verdünnt, wodurch ein Test-Präparat hergestellt wurde.

*Test-Verfahren*

Reispflanzen (Varietät: Asahi) wurden in überfluteten Porzellan-Töpfen mit einem Durchmesser von 12 cm, jeweils drei Setzlinge pro Topf, kultiviert. Im Frühstadium des Austreibens der Reispflanzen wurde die wie oben auf eine vorher festgelegte Konzentration gebrachte Test-Verbindung in den angegebenen Dosierungen mit Hilfe einer Pipette auf die Wasseroberfläche aufgebracht, so dass sie nicht unmittelbar mit den oberirdischen Teilen der Reispflanzen in Berührung kam. Vier Tage später wurde eine Suspension von Sporen des Brusone-Pilzes (Piricularia oryzae) auf die Reispflanzen gesprüht, um sie mit dem Pilz zu inokulieren. Die Pflanzen wurden 24 h in einem Inkubator bei 23-25° C und einer relativen Luftfeuchtigkeit von 100% gehalten. Danach wurden sie in ein auf 20-28° C gehaltenes Glas-Gewächshaus überführt. Sieben Tage nach der Beimpfung wurden die Pflanzen untersucht und aufgrund des folgenden Bewertungsmassstabs beurteilt. Der Bekämpfungs-Index (%) wurde berechnet.

| Grad der Erkrankung | Fläche der Verletzungen (%) |
|---|---|
| 0 | 0 |
| 0,5 | 2 oder weniger |
| 1 | 3 bis 5 |
| 2 | 6 bis 10 |
| 3 | 11 bis 20 |
| 4 | 21 bis 40 |
| 5 | 41 oder mehr |

$$\text{Bekämpfungs-Index (\%)} = \frac{\text{Grad der Erkrankung einer unbehandelten Fläche} - \text{Grad der Erkrankung einer behandelten Fläche}}{\text{Grad der Erkrankung einer unbehandelten Fläche}} \times 100$$

Die Ergebnisse sind in der Tabelle 1 aufgeführt.
Diese Erbegnisse wurden aus drei Töpfen pro Prüfung erhalten.

*Tabelle 1*

| Beispiel Nr. | Wirkstoff-Konzentration g/m² | Bekämpfungs-Index (%) | Phyto-toxizität |
|---|---|---|---|
| Verbindung d. Erfindung | 0,2 | 100 | — |
| D-1 | 0,8 | 100 | — |
| D-2 | 0,8 | 100 | — |
| D-3 | 0,8 | 100 | — |
| D-4 | 0,8 | 100 | — |

Anmerkungen zu Tabelle 1:

(1) Die Verbindung gemäss der vorliegenden Erfindung ist

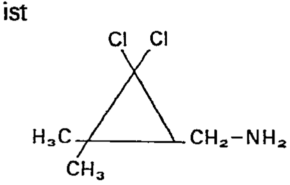

(2) Die Verbindungen D-1, D-2, D-3 und D-4, sind die gleichen, wie sie im Vorstehenden bezeichnet sind.

(3) Das Zeichen «–» in der Spalte der Phytotoxizität gibt an, dass keine Phytotoxizität vorlag.

Die in der vorliegenden Schrift beschriebene Erfindung wird wie folgt zusammengefasst:

(1) 2,2-Dichloro-3,3-dimethylcyclopropylmethylamin.

(2) Verfahren zur Herstellung von 2,2-Dichloro-3,3-dimethylcyclopropylmethylamin, bei dem eine Verbindung der nachstehenden Formel

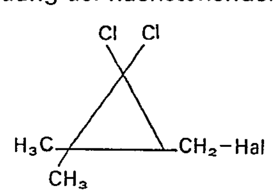

in der Hal für ein Halogen-Atom steht, mit Ammoniak umgesetzt wird.

**Patentansprüche**

1. 2,2-Dichloro-3,3-dimethylcyclopropylmethylamin der Formel (I)

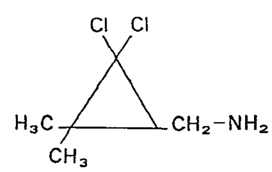 (I)

2. Verfahren zur Herstellung von 2,2-Dichloro-3,3-dimethylcyclopropylmethylamin der Formel (I),

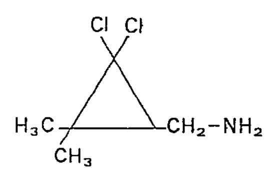 (I)

dadurch gekennzeichnet, dass ein 2,2-Dichloro-3,3-dimethylcyclopropylmethylhalogenid der Formel (II)

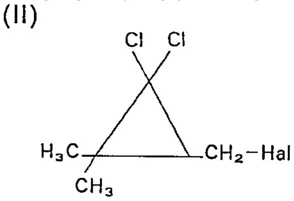 (II)

in der Hal ein Halogen-Atom bezeichnet, mit Ammoniak umgesetzt wird.

**Claims**

1. 2,2-Dichloro-3,3-dimethylcyclopropylmethylamine of the formula (I)

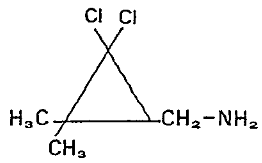

(I)

2. Process for the preparation of 2,2-dichloro-3,3-dimethyl-cyclopropylmethylamine of the formula (I)

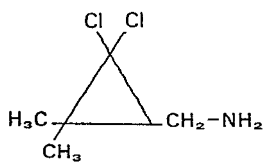

(I)

characterised in that a 2,2-dichloro-3,3-dimethylcyclopropylmethyl halide of the formula (II)

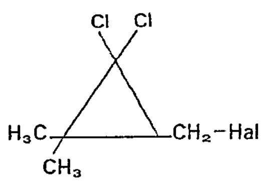

(II)

in which Hal designates a halogen atom, is reacted with ammonia.

**Revendications**

1. 2,2-dichloro-3,3-diméthylcyclopropylméthylamine de formule (I):

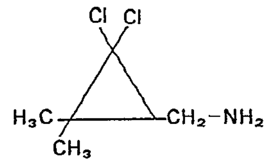

(I)

2. Procédé de fabrication de 2,2-dichloro-3,3-diméthylcyclopropylméthylamine de formule (I):

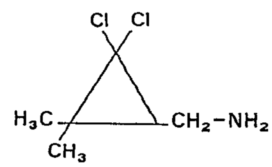

(I)

caractérisé en ce qu'on fait réagir un halogénure de 2,2-dichloro-3,3-diméthylcyclopropylméthyle de formule (II):

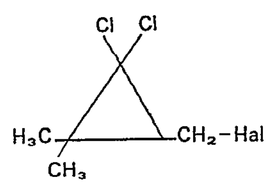

(II)

dans laquelle Hal désigne un atome d'halogène, avec de l'ammoniac.